# EUROPEAN PATENT SPECIFICATION

(11) **EP 2 519 518 B1**
(45) Date of publication and mention of the grant of the patent: **23.04.2014**
(21) Application number: 10841240.4
(22) Date of filing: 28.12.2010
(51) Int. Cl.: C07D 403/14, C07D 403/02, A61K 31/496, A61P 35/00

(54) **IMATINIB DICHLOROACETATE AND ANTI-CANCER AGENT COMPRISING THE SAME**
IMATINIB-DICHLORACETAT UND ANTIKREBSMITTEL DAMIT
DICHLOROACÉTATE D'IMATINIB ET AGENT ANTICANCÉREUX LE COMPRENANT

(30) Priority: 28.12.2009 KR 20090131719
(43) Date of publication of application: 07.11.2012
(73) Proprietor: Celltrion Chemical Research Institute, Hwaseong-si, Gyeonggi-do 445-743 (KR); Celltrion Pharm, Inc., Dongdaemun-gu, Seoul 130-100 (KR)
(72) Inventor: KIM, Kyoung Soo, Yongin-si Gyeonggi-do 446-908 (KR); PARK, Young Jun, Suwon-si Gyeonggi-do 443-813 (KR); SONG, Hyun-Nam, Hwaseong-si Gyeonggi-do 445-360 (KR); KIM, Joon Woo, Hwaseong-si Gyeonggi-do 445-897 (KR)
(74) Representative: Jansen, Cornelis Marinus
(86) International application number: PCT/KR2010/009423
(87) International publication number: WO 2011/081408

(56) References cited:
- WO-A1-2008/096987
- WO-A2-2007/019255
- WO-A2-2007/136510
- WO-A2-2009/004322
- WO-A2-2009/065910
- KR-A- 20080 044 841
- KR-B1- 100 799 821
- E D MICHELAKIS ET AL: "Dichloroacetate (DCA) as a potential metabolic-targeting therapy for cancer", BRITISH JOURNAL OF CANCER, vol. 99, no. 7, 7 October 2008 (2008-10-07), pages 989-994, XP55059872, ISSN: 0007-0920, DOI: 10.1038/sj.bjc.6604554
- BASTIN R J ET AL: "Salt Selection and Optimisation for Pharmaceutical New Chemical Entities", ORGANIC PROCESS RESEARCH AND DEVELOPMENT, CAMBRIDGE, GB, vol. 4, no. 5, 1 January 2000 (2000-01-01) , pages 427-435, XP002228592, DOI: 10.1021/OP000018U
- BYRN S ET AL: "PHARMACEUTICAL SOLIDS: A STRATEGIC APPROACH TO REGULATORY CONSIDERATIONS", PHARMACEUTICAL RESEARCH, KLUWER ACADEMIC PUBLISHERS, NEW YORK, NY, US, vol. 12, no. 7, 1 July 1995 (1995-07-01), pages 945-954, XP000996386, ISSN: 0724-8741, DOI: 10.1023/A:1016241927429

## Description

### Technical Field

The present invention relates to imatinib dichloroacetate and an anti-cancer agent comprising the same. More particularly, the present invention relates to imatinib dichloroacetate capable of inhibiting tyrosine kinase as well as inducing cancer cells to kill themselves via apoptosis, thereby inhibiting growth of cancer cells and leading to their destruction, and showing significantly enhanced anti-cancer effects by synergy between imatinib and dichloroacetic acid, and an anti-cancer agent comprising the same.

### Background Art

Imatinib is a generic name of
4-[(4-methyl-1-piperazinyl)methyl]-N-[4-methyl-3-[[4-(3-pyridyl)-2-pyrimidinyl]amin o]phenyl]benzamide of the following formula (II), which is described in U.S. Patent No. 5,521,184, which is incorporated in its entirety here by reference.

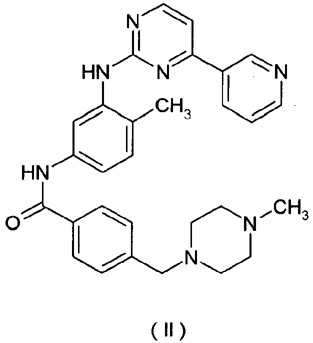

Imatinib has been developed as the first member of a class of targeted therapies, which selectively acts on leukemia cells having abnormal chromosomes, Philadelphia chromosomes (chromosomal mutants resulted from a reciprocal translocation between chromosomes 9 and 22), rather than acting on normal cells, and inhibits the growth of cancer cells.

Imatinib functions as a specific inhibitor of tyrosine kinase which is a protein useful for treating various cancers, and is used in treating chronic myelogenous leukemia, gastro-intestinal stromal tumour (GIST), solid tumor and dermatofibrosarcoma pro-tuberans, relapsed or refractory Philadelphia chromosome positive acute lymphoblastic leukemia (Ph+ ALL), myelodysplastic/myeloproliferative diseases (MDS/MPD), hypereosinophilic syndrome/chronic eosinophilic leukemia (HES/CEL) and aggressive systemic mastocytosis (ASM).

Imatinib is administered to a patient as a salt with a pharmaceutically acceptable acid. Particularly, imatinib is manufactured as imatinib mesylate of the following formula (III), and is marketed in many countries under the trade name of GLIVEC or GLEEVEC.

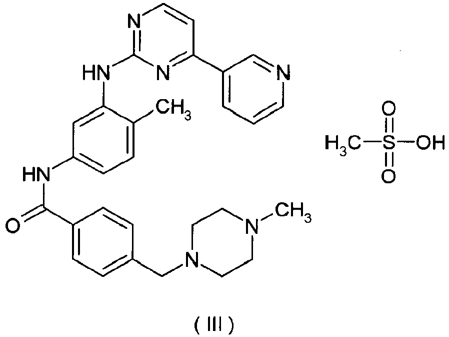

Although imatinib has been known as a useful anti-cancer drug to prolong a patient's life, it has not been reported that imatinib alone can treat cancer completely. Further, imatinib causes toleranace as other anti-cancer agents do, and has the side effects of retching, nausea, edema, rash or blood level decrease.

Therefore, a combined modality therapy administering imatinib together with a supplemental drug has been suggested to overcome the problems described above. However, the combined modality therapy also cannot destruct cancer cells completely, and has difficulties in completely and uniformly mixing two active ingredients in the same dosage unit.

Dichloroacetic acid is an acetic acid derivative in which two of the three hydrogen atoms of the methyl group have been replaced by chlorine atoms, and has activities of treating lactic acidosis and cancers. However, dichloroacetic acid is very corrosive and extremely destructive to tissues of the mucous membranes and upper respiratory tract, the salts of dichloroacetic acid such as sodium dichloroacetate and potassium dichloroacetate are therapeutically used.

Particularly, the salts of dichloroacetic acid have been used as drugs for treating lactic acidosis by inhibiting pyruvate dehydrogenase kinase and activating pyruvate dehydrogenase, which is described in Ann Intern Med 108 (1): 58∼63 (1988), which is incorporated in its entirety here by reference.

A recent study published in Cancer Cell 11 (1): 37∼51 (2007) testing dichloroacetic acid on *in vitro* cancer cell lines and a rat model, found that dichloroacetic acid restored mitochondrial function, thus restoring apoptosis, killing cancer cells *in vitro,* and shrinking the tumors in the rats.

### Disclosure of Invention

### Technical Problem

The present inventors have researched on imatinib in order to improve the anti-cancer effects and to reduce the side effects described above, and found that imatinib dichloroacetate, prepared from imatinib and dichloroacetic acid having low toxicity and inducing cancer cells to kill themselves via apoptosis, can show significantly enhanced anti-cancer effects. Further, the inventors have found that imatinib dichloroacetate can eliminate the difficulties in uniformly and completely mixing two ingredients in the same dosage unit in the case of the combination therapy.

Therefore, the present invention provides a novel imatinib dichloroacetate showing significantly enhanced anti-cancer effects.

The present invention further provides an anti-cancer agent comprising imatinib dichloroacetate.

### Solution to Problem

The present invention relates to imatinib dichloroacetate of the following formula (I).

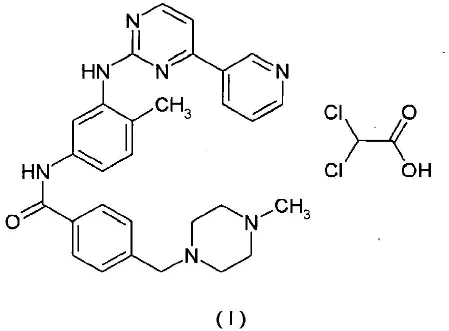

The imatinib dichloroacetate according to one embodiment of the present invention is particularly a crystalline imatinib dichloroacetate, more particularly, a crystalline imatinib dichloroacetate (hereinafter "crystalline form I") showing an X-ray powder diffraction (XRPD) pattern characterized by peaks having I/Iₒ values of at least 10% (I is the intensity of each peak; Iₒ is the intensity of the highest peak) at diffraction angles (2θ) of 5.3±0.2, 9.1±0.2, 10.5±0.2, 11.5±0.2, 12.3±0.2, 13.1±0.2, 14.1±0.2, 15.6±0.2, 16.6±0.2, 18.7±0.2, 19.8±0.2, 20.2±0.2, 20.9±0.2, 21.8±0.2, 22.5±0.2, 23.4±0.2, 24.7±0.2, 26.2±0.2, 27.9±0.2, 29.3±0.2, and 31.3±0.2.

The imatinib dichloroacetate according to another embodiment of the present invention is a crystalline imatinib dichloroacetate (hereinafter "crystalline form II") showing an X-ray powder diffraction (XRPD) pattern characterized by peaks having I/ Iₒ values of at least 10% (I is the intensity of each peak; Iₒ is the intensity of the highest peak) at diffraction angles (2θ) of 9.8±0.2, 11.0±0.2, 11.5±0.2, 13.2±0.2, 13.8±0.2, 14.7±0.2, 15.6±0.2, 16.1±0.2, 16.9±0.2, 17.4±0.2, 19.9±0.2, 20.7±0.2, 21.5±0.2, 22.4±0.2, 23.6±0.2, 24.5±0.2, 26.0±0.2, 27.2±0.2, 27.6±0.2, and 29.4±0.2.

The dichloroacetic acid used in the present invention is very safe since it has a LD₅₀ (the lethal dose causing death in 50% of rats on oral administration) of 2,820 mg/kg ( *see* Merck Index, 13 edition (2001), which is incorporated in its entirety here by reference), and has a relatively low molecular weight of 128.94 g/mol to be favorably used to give an acid addition salt. Particularly, the salts of dichloroacetic acid have been safely used to treat lactic acidosis for more than 30 years, and thus, its safety has been clinically proved.

The imatinib dichloroacetate according to the present invention can show the tyrosine kinase-inhibiting activity of imatinib, as well as the activity inducing apoptosis of cancer cells of dichloroacetic acid. Therefore, the imatinib dichloroacetate can be used as an effective drug for treating various cancers including leukemia.

The imatinib dichloroacetate of the above formula (I) according to the present invention can be prepared by reacting imatinib of the following formula (II) and dichloroacetic acid of the following formula (IV).

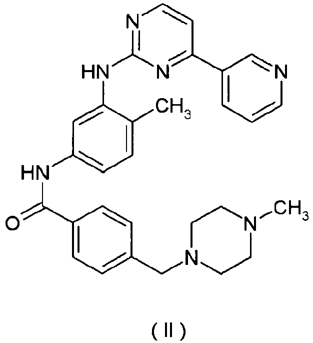

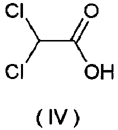

The process for preparing the imatinib dichloroacetate of the present invention is described in more detail below.

The imatinib dichloroacetate of the present invention can be prepared by suspending imatinib in an organic solvent and adding dropwise dichloroacetic acid to the resulting supspension, followed by stirring. In one embodiment, the process may further optionally include the step of:
(i) evaporating the reaction solution under reduced pressure and adding a precipitating solvent to the obtained residue, followed by stirring, and filtering the solid formed; or
(ii) stirring the reaction solution and filtering the solid formed.

In one embodiment, the dichloroacetic acid may be used in an amount of approximately 1 equivalent based on the amount of imatinib.

In one embodiment, the organic solvent may include one or more selected from alcohols such as methanol, ethanol, isopropanol, 1-butanol and hexanol; ethers such as tetrahydrofuran, dioxane, diethyl ether and diisopropyl ether; nitriles such as acetonitrile; ketones such as acetone and 2-butanone; and esters such as ethyl acetate and isopropyl acetate.

In one embodiment, the precipitating solvent may include one or more selected from alcohols such as isopropanol, 1-butanol and hexanol; ethers such as tetrahydrofuran, dioxane, diethyl ether and diisopropyl ether; nitriles such as acetonitrile; ketones such as acetone and 2-butanone; hydrocarbons such as n-pentane and n-hexane; aromatic hydrocarbons such as benzene, toluene and xylene; esters such as ethyl acetate and isopropyl acetate; and chlorinated hydrocarbons such as dichloromethane, chloroform and 1,2-dichloroethane.

In one embodiment, the reaction time may be, for example, 1 to 5 hours, and the reaction temperature may be, for example, 10 to 40 °C.

In one embodiment, the process for preparing the imatinib dichloroacetate may further include washing and drying the solid obtained after filtering.

The present invention further relates to an anti-cancer agent comprising the imatinib dichloroacetate together with a pharmaceutically acceptable carrier. In particular, the anti-cancer agent of the present invention can be used for treating chronic myelogenous leukemia or gastro-intestinal stromal tumour (GIST). In one embodiment, the anti-cancer agent of the present invention may optionally include bioactive ingredients, in addition to the imatinib dichloroacetate.

The anti-cancer agent according to the present invention can be formulated as tablets, capsules, granules, powders, emulsions, suspensions, syrups, etc. The above various forms of the anti-cancer agent can be prepared in a manner well known in the art using a pharmaceutically acceptable carrier(s) which are usually used for each form. Examples of the pharmaceutically acceptable carriers include excipient, filler, extender, binder, disintegrator, lubricant, preservative, antioxidant, isotonic agent, buffer, coating agent, sweetening agent, dissolvent, base, dispersing agent, wetting agent, suspending agent, stabilizer, colorant, flavoring agent, etc.

In one embodiment, the anti-cancer agent of the present invention may contain 1 to 90 wt%, particularly 5 to 30 wt% of the inventive imatinib dichloroacetate depending on the form thereof.

The particular dosage of the present anti-cancer agent can be varied with species of mammals including a human-being, administration route, body weight, gender, age, severity of disease, judgment of doctor, etc. By way of examples, 1 to 30 mg of the active ingredient is administered per kg of body weight a day for oral use. The total daily dosage can be administered once or over several times depending on the severity of disease, judgment of doctor, etc.

### Advantageous Effects of Invention

The imatinib dichloroacetate of the present invention can inhibit tyrosine kinase as well as induce cancer cells to kill themselves via apoptosis, thereby inhibit growth of cancer cells and lead to their destruction. Further, the imatinib dichloroacetate can increase anti-cancer effects by synergy between imatinib and dichloroacetic acid. Still further, since the imatinib dichloroacetate can have the same or higher clinical efficacy at a lower dosage due to the synergy effect, the side effects can be decreased.

Also, the imatinib dichloroacetate in accordance with the present invention eliminated the difficulties in uniformly and completely mixing imatinib and dichloroacetic acid in the same dosage unit.

Also, the crystalline imatinib dichloroacetate in accordance with the present invention has good moisture and thermal stability, and low hygroscopicity. Thus, the crystalline imatinib dichloroacetate can be useful in preparing a pharmaceutical composition.

Accordingly, the imatinib dichloroacetate in accordance with the present invention can be effectively used for preparing an anti-cancer agent for various cancers such as chronic myelogenous leukemia or gastro-intestinal stromal tumour (GIST).

### Brief Description of Drawings

Fig. 1 is an X-ray powder diffraction (XRPD) pattern of the imatinib dichloroacetate (crystalline form I) obtained in Example 1.
Fig. 2 is a differential scanning calorimeter (DSC) thermogram of the imatinib dichloroacetate (crystalline form I) obtained in Example 1.
Fig. 3 is an X-ray powder diffraction (XRPD) pattern of the imatinib dichloroacetate (crystalline form 11) obtained in Example 5.
Fig. 4 is a differential scanning calorimeter (DSC) thermogram of the imatinib dichloroacetate (crystalline form II) obtained in Example 5.
Fig. 5 is a diagram illustrating the absorbance values (%) of the solutions to which 100 nM, 10 nM and 1 nM of the imatinib dichloroacetate (crystalline form I) were added respectively, based on 100% of the absorbance value for the control.
Fig. 6 is a diagram illustrating the absorbance values (%) of the solutions to which 100 nM, 10 nM and 1 nM of the imatinib methanesulfonate (β crystalline form) were added respectively, based on 100% of the absorbance value for the control.
Fig. 7 is a diagram illustrating the absorbance values (%) of the solutions to which 100 nM, 10 nM and 1 nM of the dichloroacetic acid were added respectively, based on 100% of the absorbance value for the control.

### Best Mode for Carrying out the Invention

The present invention is further illustrated by the following examples, which are not to be construed to limit the scope of the invention.

### Example 1: Preparation of imatinib dichloroacetate (crystalline form I)

5.00 g (10.1 mmol) of imatinib was suspended in 50 ml of methanol and 0.83 ml (10.1 mmol) of dichloroacetic acid was added dropwise. Then, the reaction solution was stirred for 1 hour at 20 to 25 °C. The reaction solution was distilled under reduced pressure to be completely concentrated. 100 ml of acetone was added to the residue and the resulting solution was stirred for 2 hours at 20 to 25 °C.

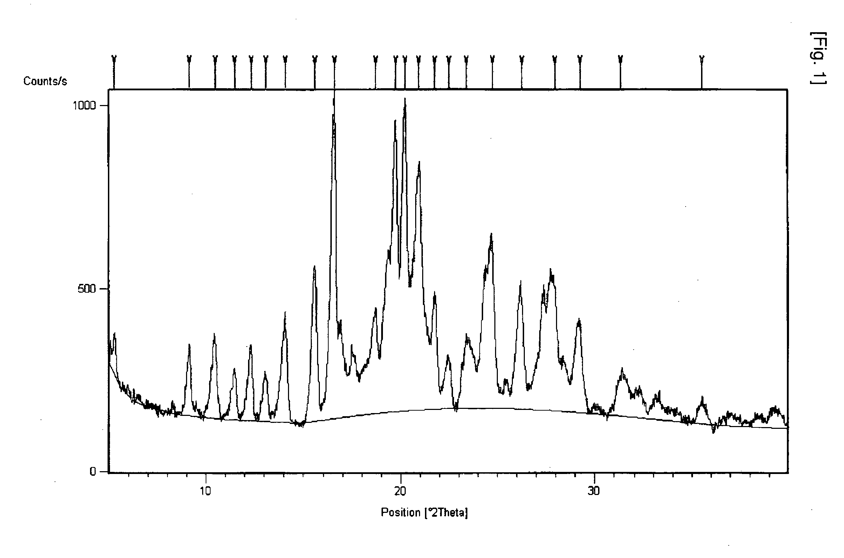

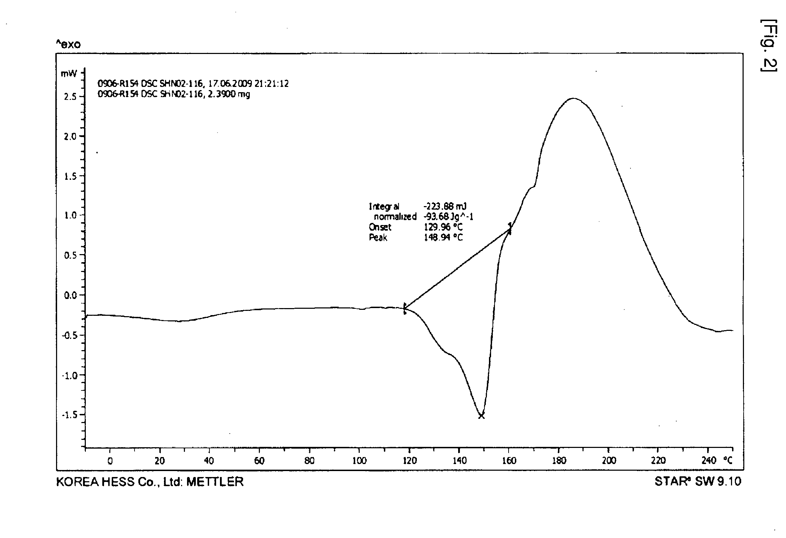

The light yellow crystalline solid formed was filtered, washed with 10 ml of acetone and dried under vacuum at 50 °C for 24 hours to give 6.10 g of the target compound. The yield was 96.7%. The obtained crystalline imatinib dichloroacetate was subjected to X-ray powder diffraction (XRPD) and differential scanning calorimeter (DSC) analyses and the results are shown in Figs. 1 and 2, respectively.
M.P.: 142-146 °C
¹H NMR (400 MHz, DMSO-d₆) : δ= 10.18 (s, 1 H), 9.27 (d, 1 H, J = 2.0 Hz), 8.94 (s, 1 H), 8.65 (dd, 1 H, J = 1.6 Hz, 4.8 Hz), 8.47 (d, 1 H, J = 4.8 Hz), 8.45 ∼ 8.42 (m, 1 H), 8.06 (d, 1 H, J = 2.0 Hz), 7.91 (d, 1 H, J = 8.4 Hz), 7.50 ∼ 7.38 (m, 5 H), 7.12 (d, 1 H, J = 8.4 Hz), 6.0 (s, 1 H), 3.60 (s, 2 H), 3.06 (brs, 4 H), 2.65 (s, 3 H), 2.60 (brs, 4 H), 2.19 (s, 3 H).

### Example 2: Preparation of imatinib dichloroacetate (crystalline form I)

5.00 g (10.1 mmol) of imatinib was suspended in 60 ml of ethanol and 0.83 ml (10.1 mmol) of dichloroacetic acid was added dropwise. Then, the reaction solution was stirred for 3 hours at 20 to 25 °C. The light yellow crystalline solid formed was filtered, washed with 10 ml of cooled ethanol and dried under vacuum at 50 °C for 24 hours to give 5.55 g of the target compound. The yield was 88.0%.

### Example 3: Preparation of imatinib dichloroacetate (crystalline form I)

5.00 g (10.1 mmol) of imatinib was suspended in 60 ml of isopropanol and 0.83 ml (10.1 mmol) of dichloroacetic acid was added dropwise. Then, the reaction solution was stirred for 3 hours at 20 to 25 °C. The light yellow crystalline solid formed was filtered, washed with 10 ml of isopropanol and dried under vacuum at 50 °C for 24 hours to give 6.30 g of the target·compound. The yield was 100.0%.

### Example 4: Preparation of imatinib dichloroacetate (crystalline form I)

5.00 g (10.1 mmol) of imatinib was suspended in 50 ml of methanol and 0.83 ml (10.1 mmol) of dichloroacetic acid was added dropwise. Then, the reaction solution was stirred for 1 hour at 20 to 25 °C. The reaction solution was distilled under the reduced pressure to be completely concentrated. 125 mℓ of dichloromethane was added to the residue and the resulting solution was stirred for 2 hours at 20 to 25 °C. The light yellow crystalline solid formed was filtered, washed with 10 ml of dichloromethane and dried under vacuum at 50 °C for 24 hours to give 6.05 g of the target compound. The yield was 96.0%.

### Example 5: Preparation of imatinib dichloroacetate (crystalline form II)

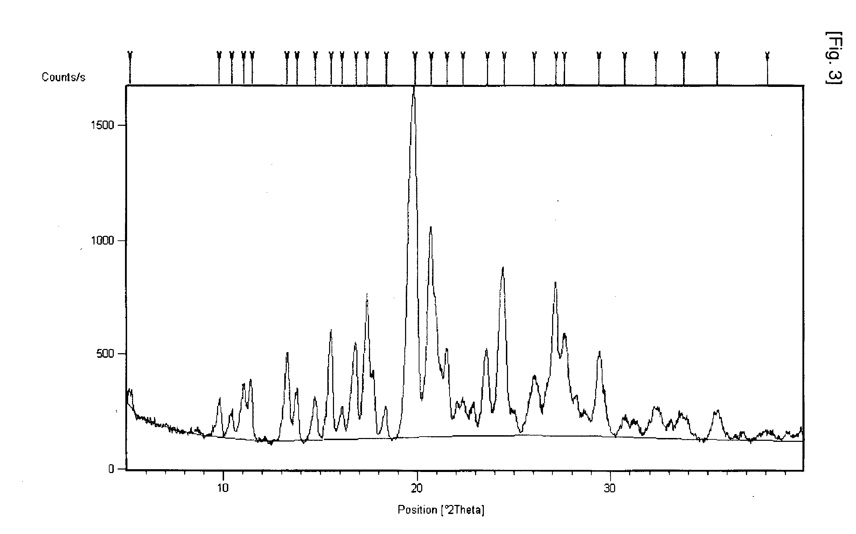

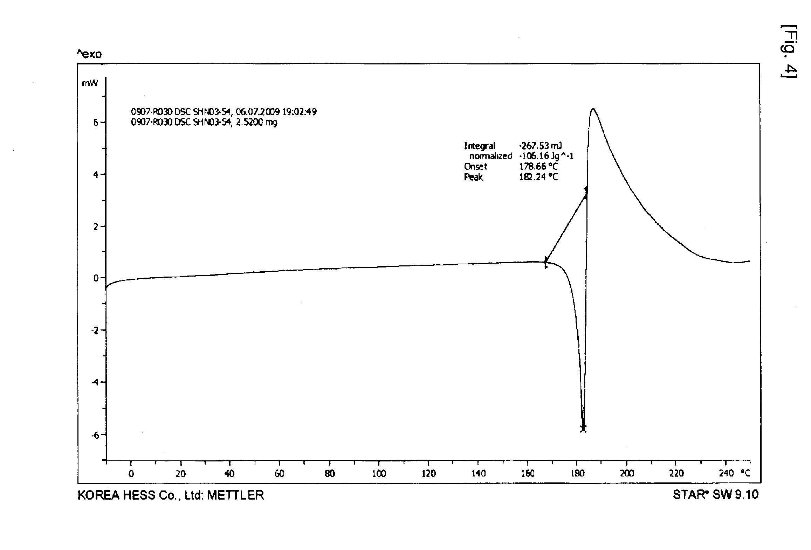

5.00 g (10.1 mmol) of imatinib was suspended in 70 ml of 2-butanone and 0.83 ml (10.1 mmol) of dichloroacetic acid was added dropwise. Then, the reaction solution was strongly stirred for 3 hours at 20 to 25 °C. The light yellow crystalline solid formed was filtered, washed with 10 ml of 2-butanone and dried under vacuum at 50 °C for 24 hours to give 6.11 g of the target compound. The yield was 96.9%. The obtained crystalline imatinib dichloroacetate was subjected to X-ray powder diffraction (XRPD) and differential scanning calorimeter (DSC) analyses and the results are shown in Figs. 3 and 4, respectively.
M.P.: 176-178 °C
¹H NMR (400MHz, DMSO-d₆) : δ= 10.18 (s, 1 H), 9.27 (d, 1 H, J = 2.0 Hz), 8.94 (s, 1 H), 8.5 (dd, 1 H, J = 1.6 Hz, 4.8 Hz), 8.47 (d, 1 H, J = 4.8 Hz), 8.45 ∼ 8.42 (m, 1 H), 8.06 (d, 1 H, J = 2.0 Hz), 7.91 (d, 1 H, J = 8.4 Hz), 7.50 ∼ 7.38 (m, 5 H), 7.12 (d, 1 H, J = 8.4 Hz), 6.0 (s, 1 H), 3.60 (s, 2 H), 3.06 (brs, 4 H), 2.65 (s, 3 H), 2.60 (brs, 4 H), 2.19 (s, 3 H)

### Example 6: Preparation of imatinib dichloroacetate (crystalline form II)

5.00 g (10.1 mmol) of imatinib was suspended in 50 ml of methanol and 0.83 ml (10.1 mmol) of dichloroacetic acid was added dropwise. Then, the reaction solution was strongly stirred for 1 hour at 20 to 25 °C. The reaction solution was distilled under reduced pressure to be completely concentrated. 70 ml of ethyl acetate was added to the residue and the resulting solution was stirred for 2 hours at 20 to 25 °C. The light yellow crystalline solid formed was filtered, washed with 10 ml of ethyl acetate and dried under vacuum at 50 °C for 24 hours to give 6.20 g of the target compound. The yield was 98.3%.

### Example 7: Preparation of imatinib dichloroacetate (crystalline form II)

5.00 g (10.1 mmol) of imatinib was suspended in 50 ml of methanol and 0.83 ml (10.1 mmol) of dichloroacetic acid was added dropwise. Then, the reaction solution was strongly stirred for 1 hour at 20 to 25 °C. The reaction solution was distilled under reduced pressure to be completely concentrated. 100 ml of acetonitrile was added to the residue and the resulting solution was stirred for 2 hours at 20 to 25 °C. The light yellow crystalline solid formed was filtered, washed with 20 ml of acetonitrile and dried under vacuum at 50 °C for 24 hours to give 5.82 g of the target compound. The yield was 92.1%.

### Example 8: Preparation of imatinib dichloroacetate (crystalline form II)

5.00 g (10.1 mmol) of imatinib was suspended in 50 ml of methanol and 0.83 ml (10.1 mmol) of dichloroacetic acid was added dropwise. Then, the reaction solution was strongly stirred for 1 hour at 20 to 25 °C. The reaction solution was distilled under reduced pressure such that the volume of the solution was concentrated to 1/3. Then, 75 ml of diethyl ether was added to the concentrated solution and the resulting solution was stirred for 2 hours at 20 to 25 °C. The light yellow crystalline solid formed was filtered, washed with 10 ml of diethyl ether and dried under vacuum at 50 °C for 24 hours to give 5.92 g of the target compound. The yield was 93.7%.

### Comparative Example 1: Preparation of imatinib methanesulfonate (β crystalline form)

Imatinib methanesulfonate (β crystalline form) was prepared in accordance with the process described in U.S. Patent No. 6,894,051, which is incorporated in its entirety here by reference.

5.0 g (10.1 mmol) of imatinib was suspended in 48 ml of methanol and 0.97 g (10.1 mmol) of methanesulfonic acid and 2 ml of methanol were added. Then, the reaction solution was heated to 50 °C, 0.5 g of active carbon was added to the reaction solution, and the solution was refluxed for 30 minutes. The reaction solution was filtered and distilled under reduced pressure, and the residue was dissolved in 15 ml of methanol. Imatinib methanesulfonate (β crystalline form) was added as a seed crystal to the resulting solution to induce the crystallization of the product. The product was dried under vacuum at 60 °C for 24 hours to give 5.18 g of the target compound.

### Experimental Example 1: X-ray structure analysis of crystalline imatinib dichloroacetate

As shown in Figs. 1 and 3, the crystalline imatinib dichloroacetate (crystalline form I) and imatinib dichloroacetate (crystalline form II) obtained in Examples 1 and 5, respectively, have distinctively characteristic peaks in the X-ray powder diffraction (XRPD) patterns. The observed characteristic peaks shown in the XRPD patterns of Figs. 1 and 3 are listed in Tables 1 and 2, respectively, wherein '2θ' is diffraction angle, 'd' is interplanar spacing, and 'I/I₀' is relative intensity of the peak.

Table 1

**[Table 1]**

| 2θ | d | I/I₀ | 2θ | d | I/I₀ |
|---|---|---|---|---|---|
| 5.2608 | 16.79867 | 13.64 | 20.2366 | 4.38827 | 95.70 |
| 9.1361 | 9.67988 | 20.89 | 20.9346 | 4.24352 | 74.63 |
| 10.4593 | 8.45812 | 25.11 | 21.7629 | 4.08383 | 34.90 |
| 11.4814 | 7.70731 | 14.66 | 22.4859 | 3.95414 | 14.56 |
| 12.3176 | 7.18591 | 23.54 | 23.3982 | 3.80199 | 20.81 |
| 13.0820 | 6.76771 | 13.65 | 24.7328 | 3.59977 | 51.20 |
| 14.0894 | 6.28598 | 30.12 | 26.2410 | 3.39620 | 35.05 |
| 15.6053 | 5.67862 | 46.99 | 27.9306 | 3.19448 | 38.76 |
| 16.5786 | 5.34738 | 100.00 | 29.2558 | 3.05272 | 27.00 |
| 18.7206 | 4.74007 | 32.37 | 31.3269 | 2.85547 | 11.78 |
| 19.7656 | 4.49176 | 87.40 | 35.5271 | 2.52692 | 6.74 |

Table 2

**[Table 2]**

| 2θ | d | I/I₀ | 2θ | d | I/I₀ |
|---|---|---|---|---|---|
| 5.1954 | 17.00996 | 6.64 | 20.6841 | 4.29434 | 64.39 |
| 9.7987 | 9.02680 | 11.30 | 21.5340 | 4.12673 | 26.78 |
| 10.4203 | 8.48968 | 7.61 | 22.3620 | 3.97577 | 11.14 |
| 11.0411 | 8.01369 | 17.08 | 23.5949 | 3.77074 | 26.11 |
| 11.4516 | 7.72732 | 15.76 | 24.4588 | 3.63948 | 48.99 |
| 13.2412 | 6.68669 | 26.52 | 26.0240 | 3.42403 | 17.86 |
| 13.7812 | 6.42588 | 15.34 | 27.1628 | 3.28301 | 47.44 |
| 14.7232 | 6.01681 | 12.54 | 27.6139 | 3.23039 | 30.67 |
| 15.5575 | 5.69596 | 31.34 | 29.3808 | 3.04003 | 25.06 |
| 16.1223 | 5.49765 | 10.15 | 30.7176 | 2.91070 | 6.12 |
| 16.8639 | 5.25755 | 27.23 | 32.3074 | 2.77101 | 9.20 |
| 17.4161 | 5.09205 | 44.40 | 33.7568 | 2.65528 | 6.84 |
| 18.3939 | 4.82351 | 9.66 | 35.4682 | 2.53098 | 8.60 |
| 19.884 | 4.46529 | 100.00 | 38.0799 | 2.36319 | 2.56 |

### Experimental Example 2: Moisture and thermal stability test

The moisture and thermal stability of an active ingredient in a pharmaceutical composition is an important factor on the perspective of the production process and long-term storage of the pharmaceutical composition. Thus, the moisture and thermal stabilities of the crystalline imatinib dichloroacetate (crystalline form I) and imatinib dichloroacetate (crystalline form II) respectively obtained in Examples 1 and 5 and the imatinib methanesulfonate (β crystalline form) obtained in Comparative Example 1 were measured.

Particularly, each imatinib acid addition salt was stored in a sealed state under an accelerated condition (a temperature of 40 °C and a relative humidity of 75 %), and after 0 (zero), 3, 7, 14 and 28 days, the remaining rate of the active ingredient was analyzed with a high performance liquid chromatography (HPLC). The results are listed in Table 3.

Table 3

**[Table 3]**

| Salts | Initial | 3 days | 7 days | 14 days | 28 days |
|---|---|---|---|---|---|
| Imatinib methanesulfonate (β crystalline form) | 100.0 | 100.1 | 100.1 | 100.0 | 99.1 |
| Imatinib dichloroacetate (crystalline form I) | 100.0 | 100.1 | 100.1 | 100.1 | 100.1 |
| Imatinib dichloroacetate (crystalline form II) | 100.0 | 100.1 | 99.9 | 99.9 | 99.8 |

As shown in Table 3, the imatinib dichloroacetate (crystalline form I) and the imatinib dichloroacetate (crystalline form II) showed the same or higher level of stability even when exposed to the accelerated condition for 28 days, as compared with the known imatinib methanesulfonate (β crystalline form). Such a result suggests that the crystalline imatinib dichloroacetate of the present invention has good chemical stability to be useful for a pharmaceutical composition.

### Experimental Example 3: Hygroscopicity test

The hygroscopicity of the imatinib dichloroacetates (crystalline forms I and II) respectively obtained in Examples 1 and 5 was compared to that of the known imatinib methanesulfonate (β crystalline form). Each compound was exposed to the condition of a temperature of 40 °C and a relative humidity of 75 % for 2 hours, 8 hours, 24 hours and 3 days, and then the amount of moisture contained in the compound was measured with Karl-Fischer titrator. The percentage amounts (weight %) of moisture contained in the active ingredients are listed in Table 4.

Table 4

**[Table 4]**

| Salts | Initial | 2 hours | 8 hours | 24 hours | 3 days |
|---|---|---|---|---|---|
| Imatinib methanesulfonate (β crystalline form) | 0.83 | 0.89 | 0.86 | 0.84 | 1.11 |
| Imatinib dichloroacetate (crystalline form I) | 1.14 | 1.14 | 1.18 | 1.14 | 1.26 |
| Imatinib dichloroacetate (crystalline form II) | 0.85 | 0.83 | 0.87 | 0.86 | 0.95 |

As shown in Table 4, the imatinib dichloroacetate (crystalline form I) and the imatinib dichloroacetate (crystalline form II) showed similar non-hygroscopicity even when exposed to the high humidity condition, as compared with the known imatinib methanesulfonate (β crystalline form). Such a result suggests that the crystalline imatinib dichloroacetate of the present invention has good moisture stability to be useful for a pharmaceutical composition.

### Experimental Example 4: Cytotoxicity test

Using a medium containing 10% of fetal bovine serum (FBS), Rosewell Park Memorial Institute medium (RPMI) and 1% of penicillin/streptomycin, a 2 x 10⁵ cells/ ml suspension of colorectal adenocarcinoma cells (HT29) was prepared. The prepared suspension was seeded into each well of 94 wells and cultured for 24 hours. Each sample was added to three wells and cultured (n=3).

The medium was removed from each well after 24 hours and the wells were washed with phosphate buffered saline (PBS). Each of the imatinib dichloroacetate (crystalline form I), imatinib methanesulfonate (β crystalline form) and dichloroacetic acid was dissolved in dimethylsulfoxide (DMSO) to prepare 10mM of solution, and then 100 nM, 10nM and 1nM of solution were respectively prepared by using serum free medium. 100 µℓ of each prepared solution was seeded into the wells and cultured at 37 °C for 72 hours.

10 µℓ of 3-(4,5-dimethylthiazol-2-yl)-2,5-diphenyltetrazolium bromide (MTT) was added to each sample and cultured for 4 hours. The absorbance of the solution was measured at 570 nm using Enzyme-linked immunosorbent assay (ELISA).

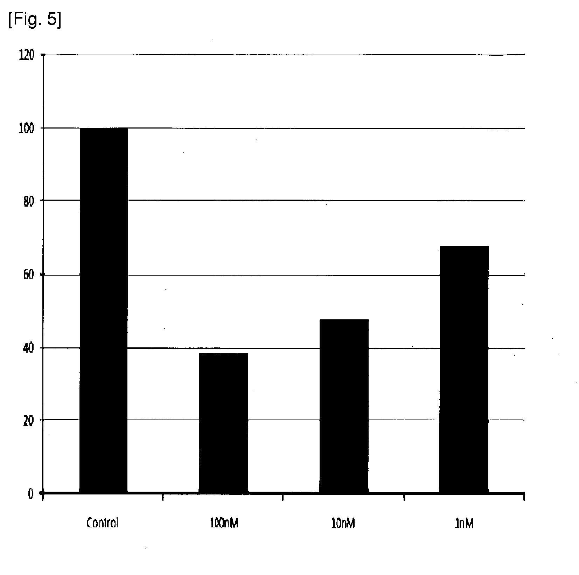

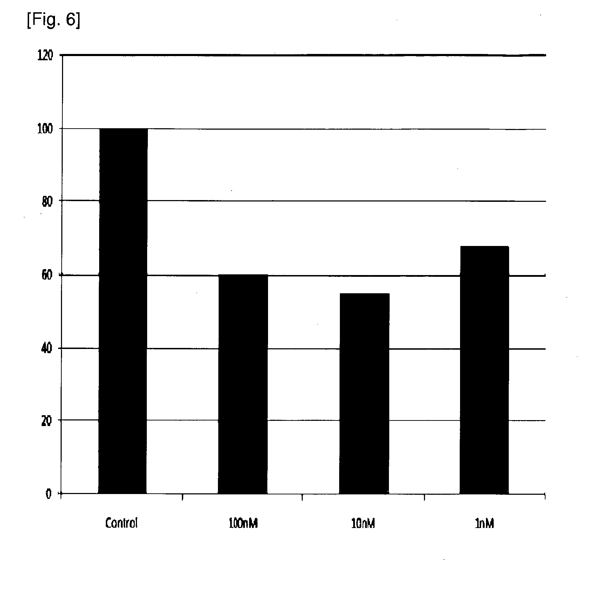

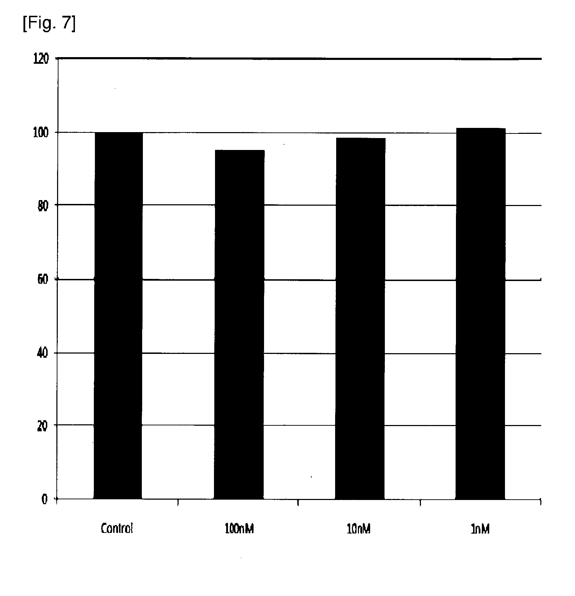

Control shows the absorbance that was measured for the serum free media containing no drug. The absorbance values of the solutions respectively treated with imatinib dichloroacetate (crystalline form I), the imatinib methanesulfonate (β crystalline form) and the dichloroacetic acid were measured and the mean of three values was calculated, as the results (%) are represented in Figs. 5, 6 and 7, respectively.

The absorbance values of the solutions to which 100 nM, 10 nM and 1 nM of the imatinib dichloroacetate (crystalline form I) were added were 38.5%, 47.8% and 67.9%, respectively, based on 100% of the absorbance value for the control.

Meanwhile, the absorbance values of the solutions to which 100 nM, 10 nM and 1 nM of the imatinib methanesulfonate (β crystalline form) were added were 59.7%, 55.2% and 67.9%, respectively. Moreover, the absorbance values of the solutions to which 100 nM, 10 nM and 1 nM of the dichloroacetic acid were added were 95.2%, 98.6% and 101.2%, respectively.

The above experimental results show that the imatinib dichloroacetate (crystalline form I) has more potent anti-cancer effects than those of the imatinib methanesulfonate (β crystalline form), and generates synergy by the addition of the dichloroacetic acid.

## Claims

1. An imatinib dichloroacetate of the following formula (I):

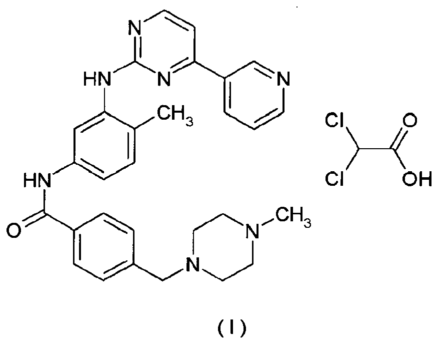

2. The imatinib dichloroacetate of Claim 1, wherein the imatinib dichloroacetate is a crystalline form.

3. The imatinib dichloroacetate of Claim 1, wherein the imatinib dichloroacetate is a crystalline form showing an X-ray powder diffraction (XRPD) pattern **characterized by** peaks having I/Iₒ values of at least 10% (I is the intensity of each peak; Iₒ is the intensity of the highest peak) at diffraction angles (2θ) of 5.3±0.2, 9.1±0.2, 10.5±0.2, 11.5±0.2, 12.3±0.2, 13.1±0.2, 14.1±0.2, 15.6±0.2, 16.6±0.2, 18.7±0.2, 19.8±0.2, 20.2±0.2, 20.9±0.2, 21.8±0.2, 22.5±0.2, 23.4±0.2, 24.7±0.2, 26.2±0.2, 27.9±0.2, 29.3±0.2, and 31.3±0.2.

4. The imatinib dichloroacetate of Claim 1, wherein the imatinib dichloroacetate is a crystalline form showing an X-ray powder diffraction (XRPD) pattern **characterized by** peaks having I/Iₒ values of at least 10% (I is the intensity of each peak; Iₒ is the intensity of the highest peak) at diffraction angles (2θ) of 9.8±0.2, 11.0±0.2, 11.5±0.2, 13.2±0.2, 13.8±0.2, 14.7±0.2, 15.6±0.2, 16.1±0.2, 16.9±0.2, 17.4±0.2, 19.9±0.2, 20.7±0.2, 21.5±0.2, 22.4±0.2, 23.6±0.2, 24.5±0.2, 26.0±0.2, 27.2±0.2, 27.6±0.2, and 29.4±0.2.

5. An anti-cancer agent comprising the imatinib dichloroacetate of any one of Claims 1 to 4 with a pharmaceutically acceptable carrier.

6. The anti-cancer agent of Claim 5, wherein the anti-cancer agent treats chronic myelogenous leukemia or gastro-intestinal stromal tumour (GIST).

## Patentansprüche

1. Imatinib-Dichloracetat der folgenden Formel (I):

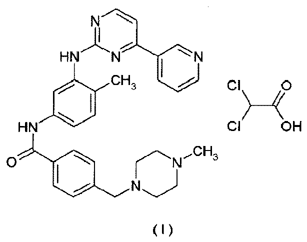

2. Imatinib-Dichloracetat nach Anspruch 1, wobei das Imatinib-Dichloracetat eine kristalline Form aufweist.

3. Imatinib-Dichloracetat nach Anspruch 1, wobei das Imatinib-Dichloracetat eine kristalline Form mit einem XRPD (X-Ray Powder Diffraction - Röntgenpulverdiffraktion)-Muster aufweist, **gekennzeichnet durch** Spitzen mit I/Iₒ-Werten von mindestens 10 % (I ist die Intensität jeder Spitze; Iₒ ist die Intensität der höchsten Spitze) bei Diffraktionswinkeln (2θ) von 5,3±0,2, 9,1+0,2, 10,5±0,2, 11,5±0,2, 12,3±0,2, 13,1+0,2, 14,1+0,2, 15,6±0,2, 16,6±0,2, 18,7±0,2, 19,8±0,2, 20,2+0,2, 20,9±0,2, 21,8±0,2, 22,5±0,2, 23,4+0,2, 24,7±0,2, 26,2+0,2, 27,9±0,2, 29,3±0,2 und 31,3±0,2.

4. Imatinib-Dichloracetat nach Anspruch 1, wobei das Imatinib-Dichloracetat eine kristalline Form mit einem XRPD (X-Ray Powder Diffraction - Röntgenpulverdiffraktion)-Muster aufweist, **gekennzeichnet durch** Spitzen mit I/Iₒ-Werten von mindestens 10 % (I ist die Intensität jeder Spitze; Iₒ ist die Intensität der höchsten Spitze) bei Diffraktionswinkeln (2 θ) von 9,8±0,2, 11,0±0,2, 11,5±0,2, 13,2±0,2, 13,8±0,2, 14,7±0,2, 15,6±0,2, 16,1±0,2, 16,9±0,2, 17,4±0,2, 19,9±0,2, 20,7±0,2, 21,5±0,2, 22,4±0,2, 23,6±0,2, 24,5±0,2, 26,0±0,2, 27,2±0,2, 27,6±0,2 und 29,4±0,2.

5. Anti-Krebs-Mittel, umfassend das Imatinib-Dichloracetat nach einem der Ansprüche 1-4 mit einem pharmazeutisch annehmbaren Träger.

6. Anti-Krebs-Mittel nach Anspruch 5, wobei das Antikrebsmittel chronische myelogene Leukämie oder gastrointestinale Stromatumore (GIST) behandelt.

## Revendications

1. Dichloroacétate d'imatinib de formule (I) suivante :

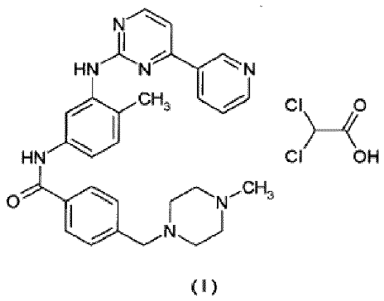

2. Dichloroacétate d'imatinib selon la revendication 1, où le dichloroacétate d'imatinib est une forme cristalline.

3. Dichloroacétate d'imatinib selon la revendication 1, où le dichloroacétate d'imatinib est une forme cristalline présentant un diagramme de diffraction des rayons X sur poudre (DRXP) **caractérisé par** des pics ayant des valeurs I/I₀ d'au moins 10 % (I est l'intensité de chaque pic ; I₀ est l'intensité du plus haut pic) à des angles de diffraction (2θ) de 5,3±0,2, 9,1±0,2, 10,5±0,2, 11,5±0,2, 12,3±0,2, 13,1±0,2, 14,1+0,2, 15,6±0,2, 16,6±0,2, 18,7±0,2, 19,8±0,2, 20,2±0,2, 20,9±0,2, 21,8±0,2, 22,5±0,2, 23,4±0,2, 24,7±0,2, 26,2±0,2, 27,9±0,2, 29,3±0,2, et 31,3±0,2.

4. Dichloroacétate d'imatinib selon la revendication 1, où le dichloroacétate d'imatinib est une forme cristalline présentant un diagramme de diffraction des rayons X sur poudre (DRXP) **caractérisé par** des pics ayant des valeurs I/I₀ d'au moins 10 % (I est l'intensité de chaque pic ; I₀ est l'intensité du plus haut pic) à des angles de diffraction (2θ) de 9,8±0,2, 11,0±0,2, 11,5±0,2, 13,2±0,2, 13,8±0,2, 14,7±0,2, 15,6±0,2, 16,1±0,2, 16,9±0,2, 17,4±0,2, 19,9±0,2, 20,7±0,2, 21,5±0,2, 22,4±0,2, 23,6±0,2, 24,5±0,2, 26,0±0,2, 27,2±0,2, 27,6±0,2, et 29,4±0,2.

5. Agent anticancéreux comprenant le dichloroacétate d'imatinib selon l'une quelconque des revendications 1 à 4 avec un vecteur pharmaceutiquement acceptable.

6. Agent anticancéreux selon la revendication 5, où l'agent anticancéreux traite la leucémie myélogène chronique ou une tumeur stromale gastro-intestinale (TSGI).
